# EUROPEAN PATENT APPLICATION

(11) **EP 4 803 618 A1**
(43) Date of publication of application: **09.09.2026**
(21) Application number: 25745285.4
(22) Date of filing: 21.01.2025
(51) Int. Cl.: C12N 9/10, C12N 15/77, C12P 19/32

(54) **NOVEL SERINE HYDROXYMETHYLTRANSFERASE VARIANT AND METHOD FOR PRODUCING PURINE NUCLEOTIDE OR GLYCINE USING SAME**

(30) Priority: 22.01.2024 KR 20240009486
(71) Applicant: CJ Cheiljedang Corporation, Seoul 04560 (KR)
(72) Inventor: KWON, Hee Su, Seoul 04560 (KR); BONG, Hyunju, Seoul 04560 (KR); RHO, Jin Ah, Seoul 04560 (KR); LEE, Ji Hye, Seoul 04560 (KR); KIM, Eunji, Seoul 04560 (KR)
(74) Representative: Meissner Bolte Partnerschaft mbB
(86) International application number: PCT/KR2025/099113
(87) International publication number: WO 2025/159620

(57) **Abstract**

The present disclosure relates to: a variant having serine hydroxymethyltransferase activity; a microorganism comprising same; a composition comprising the microorganism for producing a purine nucleotide and/or glycine; and a method for producing a purine nucleotide and/or glycine, the method comprising a step of culturing the microorganism.

## Description

### [TECHNICAL FIELD]

### Cross-Reference to Related Applications

This application claims the benefit of priority to Korean Patent Application No. 10-2024-0009486, filed on January 22, 2024, the entire contents of which are incorporated herein by reference as part of this disclosure.

This disclosure relates to a variant having serine hydroxymethyltransferase activity, a microorganism comprising the variant, a composition for producing purine nucleotides and/or glycine comprising the microorganism, and a method for producing purine nucleotides and/or glycine comprising a step of culturing the microorganism.

### [BACKGROUND ART]

Purine nucleotides serve as essential intermediates in nucleic acid biosynthetic pathways, performing vital physiological roles within the body, and are extensively utilized across the food and pharmaceutical industries. These purine nucleotides include 5'-inosine monophosphate (IMP), 5'-xanthosine monophosphate (XMP), and 5'-guanosine monophosphate (GMP), etc.

To produce purine nucleotides such as IMP, XMP, and GMP, various research has been conducted to develop high-efficiency producing microorganisms and fermentation process technologies. For example, a primary approach involves target-specific metabolic engineering, such as upregulating the expression of genes encoding enzymes involved in the biosynthesis of IMP, XMP, and GMP, or deleting genes unnecessary for the biosynthetic pathways (e.g., US 2020-0347346 A1).

However, when purine nucleotides are produced through aerobic fermentation using such microorganisms, a significant challenge arises: productivity tends to decline during the latter stages of long-term cultivation, because of an imbalance in substrate supply as the fermentation progresses in the latter stages of fermentation.

An analysis of the biosynthetic steps for microbial production of purine nucleotides reveals that carbon sources are supplied from various substrates in the biosynthetic pathway, including phosphoribosyl pyrophosphate (PRPP), glycine, and formyl-tetrahydrofolate. Among these, glycine is a key substrate for the formation of N1-(5-phospho-beta-D-ribosyl)glycinamide (GAR) by phosphoribosylamine-glycine ligase (purD), a crucial step in purine nucleotide biosynthesis. Furthermore, since glycine is also consumed in the generation of formyl-tetrahydrofolate-another essential substrate-a steady and sufficient supply of glycine is a critical factor for the efficient production of purine nucleotides.

Glycine can be synthesized through microbial metabolic processes using precursors such as serine, threonine, and glyoxylate.

When serine is used as a precursor among them, glycine is produced via a pathway starting from D-3-phosphoglycerate, involving several enzymatic steps: D-3-phosphoglycerate dehydrogenase (SerA), phosphoserine aminotransferase (SerC), and phosphoserine phosphatase (SerB), followed by the conversion of serine to glycine catalyzed by serine hydroxymethyltransferase (GlyA).

### [DISCLOSURE]

### [TECHNICAL PROBLEM]

An object of the present disclosure is to provide a polypeptide having serine hydroxymethyltransferase activity.

Another object of the present disclosure is to provide a polynucleotide encoding the polypeptide.

Another object of the present disclosure is to provide a recombinant vector comprising the polynucleotide.

Another object of the present disclosure is to provide a microorganism producing purine nucleotides and/or glycine, in which the activity of serine hydroxymethyltransferase is enhanced.

Another object of the present disclosure is to provide a microorganism comprising at least one selected from the group consisting of the polypeptide having serine hydroxymethyltransferase activity, a polynucleotide encoding the polypeptide, and a vector comprising the polynucleotide.

Another object of the present disclosure is to provide a method for producing purine nucleotides and/or glycine, comprising culturing the microorganism in a medium.

Another object of the present disclosure is to provide a composition for producing purine nucleotides and/or glycine, comprising the microorganism.

Another object of the present disclosure is to provide a use of the microorganism for producing purine nucleotides and/or glycine.

Another object of the present disclosure is to provide a use of the microorganism for the preparation of a composition for producing purine nucleotides and/or glycine.

### [TECHNICAL SOLUTION]

This can be specifically explained as follows. Meanwhile, each description and embodiment disclosed in this specification can also be applied to the other descriptions and embodiments. In other words, all combinations of the various elements disclosed herein fall within the scope of the present invention. Moreover, the scope of the present invention is not limited by the specific descriptions provided below. Additionally, throughout this specification, numerous papers and patent documents are referenced and cited. The disclosures of these cited papers and patent documents are incorporated herein by reference in their entirety, thereby clarifying the level of the technical field to which this invention pertains and providing a more detailed explanation of the contents of this invention.

An aspect of the present disclosure provides a polypeptide having serine hydroxymethyltransferase activity. The polypeptide may be a variant of serine hydroxymethyltransferase derived from a microorganism of the genus *Corynebacterium*, and may be a variant that enhances the activity of serine hydroxymethyltransferase. In one example, the polypeptide may comprise an amino acid sequence in which, in an amino acid sequence having at least 80% sequence identity to the amino acid sequence of SEQ ID NO: 31, an amino acid corresponding to the 388th residue from the N-terminus is substituted with another amino acid, an amino acid corresponding to the 409th residue from the N-terminus is substituted with another amino acid, an amino acid corresponding to the 412th residue from the N-terminus is substituted with another amino acid, or a combination thereof.

In one embodiment, the polypeptide may comprise an amino acid sequence in which, in the amino acid sequence of SEQ ID NO: 31, an amino acid corresponding to the 388th residue from the N-terminus is substituted with another amino acid, an amino acid corresponding to the 409th residue from the N-terminus is substituted with another amino acid, an amino acid corresponding to the 412th residue from the N-terminus is substituted with another amino acid, or a combination thereof.

In one example, the polypeptide may comprise an amino acid sequence in which, in an amino acid sequence having at least 80% sequence identity to the amino acid sequence of SEQ ID NO: 41, an amino acid corresponding to the 389th residue from the N-terminus is substituted with another amino acid, an amino acid corresponding to the 410th residue from the N-terminus is substituted with another amino acid, an amino acid corresponding to the 413th residue from the N-terminus is substituted with another amino acid, or a combination thereof.

In one embodiment, the polypeptide may comprise an amino acid sequence in which, in the amino acid sequence of SEQ ID NO: 41, an amino acid corresponding to the 389th residue from the N-terminus is substituted with another amino acid, an amino acid corresponding to the 410th residue from the N-terminus is substituted with another amino acid, an amino acid corresponding to the 413th residue from the N-terminus is substituted with another amino acid, or a combination thereof.

In one embodiment, the polypeptide may comprise an amino acid sequence in which, in the amino acid sequence of SEQ ID NO: 31, an amino acid corresponding to the 388th residue from the N-terminus is substituted with cysteine, an amino acid corresponding to the 409th residue from the N-terminus is substituted with arginine, or an amino acid corresponding to the 412th residue from the N-terminus is substituted with valine, or a combination thereof; and the polypeptide may comprise an amino acid sequence in which, in the amino acid sequence of SEQ ID NO: 41, an amino acid corresponding to the 389th residue from the N-terminus is substituted with cysteine, an amino acid corresponding to the 410th residue from the N-terminus is substituted with arginine, or an amino acid corresponding to the 413th residue from the N-terminus is substituted with valine, or a combination thereof.

As described above, counting amino acids from the N-terminus may mean counting with methionine (Met, M) translated from the start codon as the first amino acid.

In the present disclosure, the term "serine hydroxymethyltransferase" refers to an enzyme having an activity of catalyzing the chemical reaction of [5,10-methylenetetrahydrofolate + glycine + H₂O = tetrahydrofolate + L-serine]. For the purposes of the present application, the enzyme refers to a protein involved in producing purine nucleotides or glycine. Specifically, in the present application, "serine hydroxymethyltransferase" may be used interchangeably with the terms "SHMT" and "GlyA protein." In the present application, the sequence of the serine hydroxymethyltransferase may be obtained from GenBank of NCBI, a known database (e.g., WP_066795134.1), but is not limited thereto.

The protein to be subjected to mutation in the present application may be a wild-type protein having serine hydroxymethyltransferase activity. Specifically, the serine hydroxymethyltransferase to be subjected to mutation may be an amino acid sequence having at least 80% sequence identity to SEQ ID NO: 31, and more specifically, may comprise, consist of, or consist essentially of the amino acid sequence of SEQ ID NO: 31 or SEQ ID NO: 41, but is not limited thereto. That is, it does not exclude the addition of meaningless sequences to the front or back of the amino acid sequence of SEQ ID NO: 31 or SEQ ID NO: 41, naturally occurring mutations, or silent mutations thereof, and any protein having the same or corresponding activity as a protein comprising the amino acid sequence of SEQ ID NO: 31 or SEQ ID NO: 41 may correspond to the protein to be subjected to mutation in the present application. For example, the protein to be subjected to mutation in the present application may be a protein consisting of an amino acid sequence having at least 80%, 85%, 90%, 95%, 96%, 97%, 98%, 99%, 99.1%, 99.3%, 99.5%, 99.7%, or 99.9% or more, and less than 100% sequence homology or identity to the amino acid sequence of SEQ ID NO: 31 or SEQ ID NO: 41. In addition, as long as it is an amino acid sequence having such homology or identity and exhibiting efficacy corresponding to the protein, a protein having an amino acid sequence in which a part of the sequence is deleted, modified, substituted, or added may also be included in the scope of the protein to be subjected to mutation of the present disclosure.

In the present disclosure, the serine hydroxymethyltransferase may be derived from a microorganism of the genus *Corynebacterium*, specifically from *Corynebacterium stationis* or *Corynebacterium glutamicum,* but is not limited thereto.

An aspect of the present disclosure provides a polypeptide comprising a mutation at the 388th position and/or 409th position and/or 412th position from the N-terminus in the amino acid sequence of SEQ ID NO: 31. The polypeptide may be a variant of serine hydroxymethyltransferase. The variant of serine hydroxymethyltransferase may be one that increases the activity of serine hydroxymethyltransferase and/or the ability to produce purine nucleotides and/or glycine. The variant of serine hydroxymethyltransferase means that the 388th amino acid and/or 409th amino acid and/or 412th amino acid from the N-terminus are mutated in the above-described SEQ ID NO: 31 and/or an amino acid having at least 80%, 85%, 90%, 95%, 96%, 97%, 98%, or 99% homology or identity to SEQ ID NO: 31. The variant may be derived from *Corynebacterium stationis*, but is not limited thereto.

As an example, the variant of serine hydroxymethyltransferase may consist of a polypeptide comprising an amino acid sequence in which, in the amino acid sequence of SEQ ID NO: 31, i) an amino acid corresponding to the 388th residue is substituted with cysteine (Cys, C), ii) an amino acid corresponding to the 409th residue is substituted with arginine (Arg, R), iii) an amino acid corresponding to the 412th residue is substituted with valine (Val, V), or iv) an amino acid corresponding to the 388th residue is substituted with cysteine, an amino acid corresponding to the 409th residue is substituted with arginine, and an amino acid corresponding to the 412th residue is substituted with valine. In addition, the variant of serine hydroxymethyltransferase of the present application may comprise a polypeptide having at least 80%, 85%, 90%, 95%, 96%, 97%, 98%, or 99% homology or identity to an amino acid sequence in which, in the amino acid sequence of SEQ ID NO: 31, the 388th amino acid from the N-terminus is substituted with cysteine and/or the 409th amino acid is substituted with arginine and/or the 412th amino acid is substituted with valine. In addition, it is obvious that a protein having an amino acid sequence in which a part of the sequence is deleted, modified, substituted, or added, in addition to the amino acid sequence at the 388th, 409th, and/or 412th positions, is also included in the scope of the present disclosure, as long as it is an amino acid sequence having such homology or identity and exhibiting activity corresponding to the protein.

Another aspect of the present disclosure provides a polypeptide comprising a mutation at the 389th position and/or 410th position and/or 413th position from the N-terminus in the amino acid sequence of SEQ ID NO: 41. The polypeptide may be a variant of serine hydroxymethyltransferase. The variant of serine hydroxymethyltransferase may be one that increases the activity of serine hydroxymethyltransferase and/or the ability to produce purine nucleotides and/or glycine. The variant of serine hydroxymethyltransferase means that the 389th amino acid and/or 410th amino acid and/or 413th amino acid from the N-terminus are mutated in the above-described SEQ ID NO: 41 and/or an amino acid having at least 80%, 85%, 90%, 95%, 96%, 97%, 98%, or 99% homology or identity to SEQ ID NO: 41. The variant may be derived from *Corynebacterium glutamicum*, but is not limited thereto.

As an example, the variant of serine hydroxymethyltransferase may consist of a polypeptide comprising an amino acid sequence in which, in the amino acid sequence of SEQ ID NO: 41, i) an amino acid corresponding to the 389th residue is substituted with cysteine (Cys, C), ii) an amino acid corresponding to the 410th residue is substituted with arginine (Arg, R), iii) an amino acid corresponding to the 413th residue is substituted with valine (Val, V), or iv) an amino acid corresponding to the 389th residue is substituted with cysteine, an amino acid corresponding to the 410th residue is substituted with arginine, and an amino acid corresponding to the 413th residue is substituted with valine. In addition, the variant of serine hydroxymethyltransferase of the present application may comprise a polypeptide having at least 80%, 85%, 90%, 95%, 96%, 97%, 98%, or 99% homology or identity to an amino acid sequence in which the 389th amino acid is substituted with cysteine and/or the 410th amino acid is substituted with arginine and/or the 413th amino acid is substituted with valine from the N-terminus in the amino acid sequence of SEQ ID NO: 41. In addition, it is obvious that a protein having an amino acid sequence in which a part of the sequence is deleted, modified, substituted, or added, in addition to the amino acid sequence at the 389th, 410th, and/or 413th positions, is also included in the scope of the present application, as long as it is an amino acid sequence having such homology or identity and exhibiting activity corresponding to the protein.

In one embodiment, the variant of serine hydroxymethyltransferase may comprise or consist of an amino acid sequence having at least 70%, 75%, 80%, 85%, 90%, 95%, 96%, 96.3%, 96.5%, 96.7%, 96.9%, 97%, 97.1%, 97.2%, 97.4%, 97.6%, 97.8%, 98%, 98.2%, 98.4%, 98.6%, 98.9%, 99%, 99.1%, 99.3%, 99.5%, 99.7%, or 99.9% homology or identity to any one amino acid sequence selected from the group consisting of SEQ ID NOs: 32 to 35.

In one embodiment, the variant of serine hydroxymethyltransferase may comprise or consist of an amino acid sequence having at least 70%, 75%, 80%, 85%, 90%, 95%, 96%, 96.3%, 96.5%, 96.7%, 96.9%, 97%, 97.1%, 97.2%, 97.4%, 97.6%, 97.8%, 98%, 98.2%, 98.4%, 98.6%, 98.9%, 99%, 99.1%, 99.3%, 99.5%, 99.7%, or 99.9% homology or identity to any one amino acid sequence selected from the group consisting of SEQ ID NOs: 42 to 45.

In addition, a polypeptide having such homology or identity and exhibiting activity corresponding to the variant of serine hydroxymethyltransferase may be included in the variant of the present application even if it has an amino acid sequence in which a part of the sequence is deleted, modified, substituted, conservatively substituted, and/or added. For example, it may be a case of having a sequence addition or deletion, a naturally occurring mutation, a silent mutation, or a conservative substitution at the N-terminus, C-terminus, and/or inside of the amino acid sequence of the variant of serine hydroxymethyltransferase of the present application that does not change the activity of the variant.

The "conservative substitution" refers to substituting one amino acid with another amino acid having similar structural and/or chemical properties. Such amino acid substitutions may generally occur based on similarities in the polarity, charge, solubility, hydrophobicity, hydrophilicity, and/or amphipathic nature of the residues. Typically, a conservative substitution may have little or no effect on the activity of a protein or polypeptide.

In one example, in the amino acid sequence of SEQ ID NO: 31, an amino acid corresponding to the 388th residue may be phenylalanine (Phe, F), an amino acid corresponding to the 409th residue may be lysine (Lys, K), and an amino acid corresponding to the 412th residue may be alanine (Ala, A), but is not limited thereto.

In one example, in the amino acid sequence of SEQ ID NO: 41, an amino acid corresponding to the 389th residue may be phenylalanine (Phe, F), an amino acid corresponding to the 410th residue may be serine (Ser, S), and an amino acid corresponding to the 413th residue may be glycine (Gly, G), but is not limited thereto.

The variant of serine hydroxymethyltransferase of the present disclosure may have a property of increasing the ability to produce purine nucleotides and/or glycine compared to a wild-type polypeptide having serine hydroxymethyltransferase activity.

In the present disclosure, the term "purine nucleotide" may specifically be one or more nucleotides selected from the group consisting of 5'-inosine monophosphate (hereinafter, IMP), 5'-xanthosine monophosphate (hereinafter, XMP), and 5'-guanosine monophosphate (hereinafter, GMP). The IMP is a deaminated compound of adenine and refers to a nucleotide composed of one molecule each of hypoxanthine, ribose, and phosphoric acid. It may be biosynthesized from 5'-phosphoribosyl-1-pyrophosphate (PRPP); specifically, it may be formed by substituting the pyrophosphate group bonded to the C1 carbon of PRPP with a nitrogen atom and constructing an imidazole ring and a pyrimidine ring through 9 steps. The XMP refers to a nucleotide dehydrogenated from IMP. It may be synthesized from IMP by inosine-5'-monophosphate dehydrogenase. The GMP refers to a nucleotide having a structure in which a phosphate group forms an ester bond with the ribose moiety in a guanosine molecule. The GMP may be synthesized by adding an ammonia molecule to XMP by 5'-guanosine monophosphate synthase (GMP synthase). A method for producing GMP from XMP and/or a means used for the method may be selected from known techniques.

In the present disclosure, the term "glycine" refers to an amino acid that is a colorless crystal with a sweet taste, also named glycine, and has the chemical formula C₂H₅NO₂.

In the present disclosure, the term "production ability" refers to the ability to produce purine nucleotides and/or glycine, and may be used interchangeably with "productivity". It can be confirmed by measuring the concentration of purine nucleotides and/or glycine contained in a culture medium after culturing a strain that produces purine nucleotides and/or glycine.

In the present disclosure, the term "variant" refers to a polypeptide in which one or more amino acids are subjected to conservative substitution and/or modification, thereby differing from the amino acid sequence before mutation of the variant, but maintaining functions or properties. Such a variant can generally be identified by modifying one or more amino acids in the amino acid sequence of the polypeptide and evaluating the properties of the modified polypeptide. That is, the ability of the variant may be increased, unchanged, or decreased compared to the polypeptide before mutation. In addition, some variants may include variants in which one or more parts, such as an N-terminal leader sequence or a transmembrane domain, have been removed. Other variants may include variants in which a portion has been removed from the N- and/or C-terminus of a mature protein. The term "variant" may be used interchangeably with terms such as modification, modified polypeptide, modified protein, mutant, mutein, divergent, variant, etc. (in English expressions: modification, modified polypeptide, modified protein, mutant, mutein, divergent, variant, etc.), and is not limited thereto as long as the term is used in the sense of being mutated. For the purposes of the present application, the variant may be a polypeptide having serine hydroxymethyltransferase activity, wherein in the amino acid sequence of SEQ ID NO: 31, an amino acid corresponding to the 388th residue is substituted with cysteine, an amino acid corresponding to the 409th residue is substituted with arginine, an amino acid corresponding to the 412th residue is substituted with valine, or a combination thereof; or a polypeptide having serine hydroxymethyltransferase activity, wherein in the amino acid sequence of SEQ ID NO: 41, an amino acid corresponding to the 389th residue is substituted with cysteine, an amino acid corresponding to the 410th residue is substituted with arginine, an amino acid corresponding to the 413th residue is substituted with valine, or a combination thereof.

Additionally, variants may include deletions or additions of amino acids that have minimal impact on the properties and secondary structure of the polypeptide. For example, a signal (or leader) sequence involved in the translocation of the protein co-translationally or post-translationally may be conjugated to the N-terminus of the variant. Furthermore, the variant may be conjugated with other sequences or linkers so that it can be identified, purified, or synthesized.

In one example, the variant may be encoded by a polynucleotide comprising or consisting of any one nucleic acid sequence selected from SEQ ID NOs: 37 to 40 and SEQ ID NOs: 47 to 50.

Another embodiment of the present disclosure provides a polynucleotide encoding the polypeptide having serine hydroxymethyltransferase activity (e.g., a variant of serine hydroxymethyltransferase).

In the present disclosure, the term "polynucleotide" refers to a polymer of nucleotides in which nucleotide monomers are connected in a long chain by covalent bonds, meaning a DNA or RNA strand of a certain length or more.

The polynucleotide encoding the polypeptide having serine hydroxymethyltransferase activity (e.g., a variant of serine hydroxymethyltransferase) of the present disclosure may include, without limitation, any polynucleotide sequence encoding a polypeptide having serine hydroxymethyltransferase activity. In the present application, the gene encoding the amino acid sequence of serine hydroxymethyltransferase is the *glyA* gene, which may be derived from a microorganism of the genus *Corynebacterium*, specifically from *Corynebacterium stationis* or *Corynebacterium glutamicum*, but is not limited thereto.

In one example, the polynucleotide may comprise, consist of, or consist essentially of the nucleic acid sequence (base sequence) described in any one SEQ ID NO selected from SEQ ID NOs: 37 to 40 and SEQ ID NOs: 47 to 50.

The polynucleotide consisting of or comprising any one nucleic acid sequence selected from the group consisting of SEQ ID NOs: 37 to 40 and SEQ ID NOs: 47 to 50 may encode the amino acid sequence described in any one SEQ ID NO selected from the group consisting of SEQ ID NOs: 32 to 35 and SEQ ID NOs: 42 to 45, respectively.

In the polynucleotide of the present disclosure, various modifications may be made to the coding region within a range that does not change the amino acid sequence of the variant of the present application, in consideration of the degeneracy of codons or preferred codons in the organism in which the variant of the present application is to be expressed. Specifically, the polynucleotide of the present application may have or comprise a base sequence having at least 70%, 75%, 80%, 85%, 90%, 95%, 96%, 97%, 98%, 99%, 99.1%, 99.2%, 99.3%, 99.4%, 99.5%, 99.6%, 99.7%, 99.8%, or 99.9% homology or identity to any one base sequence selected from the group consisting of SEQ ID NOs: 37 to 40 and SEQ ID NOs: 47 to 50; or may consist of or consist essentially of a base sequence having at least 70%, 75%, 80%, 85%, 90%, 95%, 96%, 97%, 98%, 99%, 99.1%, 99.2%, 99.3%, 99.4%, 99.5%, 99.6%, 99.7%, 99.8%, or 99.9% homology or identity to any one sequence selected from the group consisting of SEQ ID NOs: 37 to 40 and SEQ ID NOs: 47 to 50, but is not limited thereto.

The polynucleotide of the present disclosure may include, without limitation, probes that can be prepared from known gene sequences, for example, sequences capable of hybridizing under stringent conditions with a complementary sequence to all or part of the polynucleotide sequence of the present application. The "stringent condition" refers to a condition that enables specific hybridization between polynucleotides. Such conditions are specifically described in the literature (refer to J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). For example, conditions include hybridizing polynucleotides with high homology or identity, such as at least 70%, 75%, 80%, 85%, 90%, 95%, 96%, 97%, 98%, 99%, 99.1%, 99.2%, 99.3%, 99.4%, 99.5%, 99.6%, 99.7%, 99.8%, or 99.9% homology or identity, while not hybridizing polynucleotides with lower homology or identity; or washing conditions for typical Southern hybridization at a salt concentration and temperature equivalent to 60°C, 1×SSC, 0.1% SDS, specifically 60°C, 0.1×SSC, 0.1% SDS, and more specifically 68°C, 0.1×SSC, 0.1% SDS, for once, specifically 2 to 3 times.

Hybridization requires that two nucleotides be complementary sequences, but hybridized polynucleotides may include some mismatches between bases depending on the stringency of hybridization. The term "complementary" is used to describe the relationship between nucleotide bases capable of hybridizing with each other. For example, regarding DNA, adenine is complementary to thymine and cytosine is complementary to guanine. Therefore, the polynucleotide of the present application may also include isolated nucleic acid fragments complementary to the entire sequence as well as substantially similar nucleic acid sequences.

Specifically, a polynucleotide having homology or identity with the polynucleotide of the present disclosure can be detected using hybridization conditions including a hybridization step at a Tm value of 55°C and using the conditions described above. Additionally, the Tm value may be 60°C, 63°C, or 65°C, but is not limited thereto and can be appropriately adjusted by those skilled in the art according to the purpose.

The appropriate stringency for hybridizing the polynucleotide depends on the length and degree of complementarity of the polynucleotide, and variables are well known in the art (e.g., J. Sambrook et al., *supra*).

In the present disclosure, saying that a polynucleotide (which may be used interchangeably with "gene") or a polypeptide (which may be used interchangeably with "protein") "comprises a specific nucleic acid sequence or amino acid sequence, consists of a specific nucleic acid sequence or amino acid sequence, or is represented by a specific nucleic acid sequence or amino acid sequence" may mean that the polynucleotide or polypeptide essentially includes the specific nucleic acid sequence or amino acid sequence, and can be interpreted as including (or not excluding insignificant variations in) "substantially equivalent sequences" where insignificant variations (deletions, substitutions, modifications, and/or additions) are made to the specific nucleic acid sequence or amino acid sequence within a range that maintains the original function and/or desired function of the polynucleotide or polypeptide.

In the present disclosure, the term "homology" or "identity" refers to the degree of mutual similarity between two given amino acid sequences or base sequences and can be expressed as a percentage. The terms homology and identity can often be used interchangeably.

The sequence homology or identity of conserved polynucleotides or polypeptides is determined by standard alignment algorithms, and default gap penalties established by the program used may be utilized together. Substantially, homologous or identical sequences can generally hybridize with the whole or a part of the sequence under intermediate or high stringent conditions. It is obvious that hybridization also includes hybridization with polynucleotides containing codons considering general codons or codon degeneracy in polynucleotides.

Whether any two polynucleotide or polypeptide sequences have homology or identity can be determined using known computer algorithms such as the "FASTA" program using default parameters as in, for example, Pearson et al (https://www.google.com/search?q=1988) [Proc. Natl. Acad. Sci. USA 85]: 2444. Alternatively, it can be determined using the Needleman-Wunsch algorithm (Needleman and Wunsch, 1970, J. Mol. Biol. 48: 443-453) as performed in the Needle program of the EMBOSS package (EMBOSS: The European Molecular Biology Open Software Suite, Rice et al., 2000, Trends Genet. 16: 276-277) (version 5.0.0 or later) (including the GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego, 1994, and CARILLO ET AL. SIAM J Applied Math 48: 1073). For example, homology or identity can be determined using BLAST of the National Center for Biotechnology Information or ClustalW.

Homology or identity of polynucleotides or polypeptides can be determined by comparing sequence information using a GAP computer program, such as, for example, Needleman et al. (1970), J Mol Biol. 48:443, as known in Smith and Waterman, Adv. Appl. Math (1981) 2:482. In summary, the GAP program can be defined as the number of similarly aligned symbols (i.e., nucleotides or amino acids) divided by the total number of symbols in the shorter of the two sequences. Default parameters for the GAP program may include (1) a binary comparison matrix (containing a value of 1 for identity and 0 for non-identity) and a weighted comparison matrix of Gribskov et al (1986) Nucl. Acids Res. 14: 6745 (or EDNAFULL (EMBOSS version of NCBI NUC4.4) substitution matrix), as disclosed by Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap opening penalty 10, gap extension penalty 0.5); and (3) no penalty for end gaps.

In the present disclosure, the term "corresponding to" refers to an amino acid residue at a position listed in a polypeptide, or an amino acid residue that is similar, identical, or homologous to a residue listed in a polypeptide. Identifying an amino acid at a corresponding position may be determining a specific amino acid in a sequence with reference to a specific sequence. The "corresponding region" used in the present application generally refers to a similar or corresponding position in a related protein or a reference protein.

For example, any amino acid sequence can be aligned with SEQ ID NO: 31, and based on this, each amino acid residue of the amino acid sequence can be numbered with reference to the numerical position of the amino acid residue corresponding to the amino acid residue of SEQ ID NO: 31. For example, a sequence alignment algorithm as described in the present application can identify the position of an amino acid or the position where a modification such as substitution, insertion, or deletion occurs compared to a query sequence (also referred to as a "reference sequence").

For such alignment, for example, the Needleman-Wunsch algorithm (Needleman and Wunsch, 1970, J. Mol. Biol. 48: 443-453), the Needle program of the EMBOSS package (EMBOSS: The European Molecular Biology Open Software Suite, Rice et al., 2000, Trends Genet. 16: 276-277), etc., may be used, but is not limited thereto, and sequence alignment programs, pairwise sequence comparison algorithms, etc., known in the art may be appropriately used.

Another aspect of the present disclosure provides a vector comprising a polynucleotide encoding the polypeptide having serine hydroxymethyltransferase activity (e.g., a variant of serine hydroxymethyltransferase). The vector may be an insertion vector or an expression vector.

In the present disclosure, the term "vector" refers to a DNA construct for delivering a target polynucleotide into a suitable host or host cell. As an example, it may include a base sequence of a polynucleotide encoding a target polypeptide operably linked to a suitable expression control region (or expression control sequence) so that the target polypeptide can be expressed in a suitable host cell, but is not limited thereto. The expression control sequence may include a promoter capable of initiating transcription, any operator sequence for controlling transcription, a sequence encoding a suitable mRNA ribosome binding site, and/or a sequence controlling the termination of transcription and/or translation. After being transformed into a suitable host cell, the vector may be maintained independently of the genome of the host cell or may be inserted into the genome of the host cell. For example, the target polynucleotide can be inserted into a chromosome through an insertion vector. The insertion of the polynucleotide into a chromosome may be performed by any method known in the art, for example, homologous recombination, but is not limited thereto.

The vector usable in the present disclosure is not particularly limited as long as it is replicable within a host cell, and may be selected from any commonly used vectors. Examples of commonly used vectors include plasmids, cosmids, viruses, and bacteriophages in their natural or recombinant state. For example, as a phage vector or cosmid vector, can be used as a phage vector or a cosmid vector, and pBR-based, pUC-based, pBluescriptII-based, pGEM-based, pTZ-based, pCL-based, and pET-based, etc. can be used as a plasmid vector. Specifically, pDZ, pACYC177, pACYC184, pCL, pECCG117, pUC19, pBR322, pMW118, pCC1BAC, pDC24 vectors, etc. can be exemplified, but are not limited thereto.

The vector may further include a selection marker for confirming whether the vector has been introduced into the transformed cell or integrated into the genome of the transformed cell. The selection marker is for confirming whether the cell transformed with the vector or the polynucleotide has been inserted and can be selected and used from genes that grant a selectable phenotype such as drug resistance, auxotrophy, resistance to cytotoxic agents, or expression of surface proteins. In an environment treated with a selective agent, only cells expressing the selection marker survive or exhibit other phenotypes, so that transformed cells can be selected.

Expressing the polypeptide (variant) in a microorganism may be performed by introducing a polynucleotide encoding the variant, or a vector comprising the same, into a host cell and culturing a recombinant cell (e.g., a microorganism) comprising the same.

The introduction of the polynucleotide encoding the polypeptide (variant) or a vector comprising the same into a microorganism may be performed by a person skilled in the art appropriately selecting a known transformation method. In the present specification, the term "transformation" refers to changing the genetic characteristics of a host cell (microorganism) by introducing a target polynucleotide or a vector comprising the same into the host cell (microorganism). The transformed polynucleotide may be positioned by being integrated into the chromosome of the host cell or may be positioned extra-chromosomally. The polynucleotide may be introduced in an appropriate form depending on the purpose of introduction. For example, the polynucleotide may be introduced into a host cell in the form of an expression cassette, which is a gene construct comprising all elements required for its own expression. The expression cassette may typically include expression control elements such as a promoter, a transcription termination signal, a ribosome-binding site, and/or a translation termination signal operably linked to the polynucleotide. The expression cassette may be in the form of an expression vector capable of self-replication. Additionally, the polynucleotide may be introduced into the host cell in its own form and operably linked to a sequence required for expression in the host cell. As used herein, the term "operably linked" may mean that an expression control element (e.g., a promoter) and a polynucleotide are functionally connected so as to perform transcriptional regulation (e.g., initiation of transcription) of the polynucleotide. Operable linkage can be performed using genetic recombination techniques known in the art.

The method of transforming the polynucleotide into a host cell can be performed by any method of introducing a nucleic acid into a cell (microorganism) and can be carried out by appropriately selecting a transformation technique known in the art depending on the host cell. Examples of such known transformation methods include electroporation, calcium phosphate (CaPO₄*)* precipitation, calcium chloride (CaCl₂) precipitation, microinjection, polyethylene glycol (PEG)-mediated uptake, DEAE-dextran method, cationic liposome method, lipofection, and lithium acetate-DMSO method, but are not limited thereto.

Another aspect of the present disclosure provides a microorganism with enhanced serine hydroxymethyltransferase activity.

The microorganism may be a microorganism comprising one or more (e.g., one or more, two or more, or one, two, or three) selected from the group consisting of the polypeptide (variant) having serine hydroxymethyltransferase activity described above, a polynucleotide encoding the polypeptide, and a vector comprising the polynucleotide.

In the present disclosure, "enhancement" of polypeptide activity (e.g., serine hydroxymethyltransferase activity) means that the activity of the polypeptide in a host cell (microorganism) is increased compared to its endogenous activity. The term enhancement may be used interchangeably with terms such as activation, up-regulation, overexpression, and increase. Here, activation, enhancement, up-regulation, overexpression, and increase may all include manifesting an activity that was not originally possessed, or manifesting improved activity compared to endogenous activity or activity before modification. The "endogenous activity" refers to the activity of a specific polypeptide originally possessed by a parent strain or an unmodified microorganism before a phenotypic change occurs due to genetic variation caused by natural or artificial factors. This may be used interchangeably with "activity before modification". That the activity of a polypeptide is "enhanced," "up-regulated," "overexpressed," or "increased" compared to the endogenous activity means that it is improved compared to the activity and/or concentration (expression level) of the specific polypeptide originally possessed by the parent strain or unmodified microorganism before the phenotypic change.

The enhancement can be achieved by introducing an exogenous polypeptide or through the enhancement of endogenous polypeptide activity and/or concentration (expression level). Whether the activity of the polypeptide is enhanced can be confirmed from the degree of activity of the polypeptide, the expression level, or an increase in the amount of product discharged from the polypeptide.

For the enhancement of the activity of the polypeptide, various methods well known in the art can be applied, and it is not limited as long as the activity of the target polypeptide can be enhanced compared to that of the microorganism before modification. Specifically, it may involve using genetic engineering and/or protein engineering well known to those skilled in the art, which are routine methods in molecular biology, but is not limited thereto (e.g., Sitnicka et al. Functional Analysis of Genes. Advances in Cell Biology. 2010, Vol. 2. 1-16; Sambrook et al. Molecular Cloning 2012, etc.).

Specifically, the enhancement of the polypeptide of the present application may be:
1) increasing the intracellular copy number of a polynucleotide encoding the polypeptide;
2) replacing a gene expression regulatory region on a chromosome encoding the polypeptide with a sequence having strong activity;
3) modification of a nucleotide sequence encoding an initiation codon or a 5'-UTR region of a gene transcript encoding the polypeptide;
4) modification of an amino acid sequence of the polypeptide such that the polypeptide activity is enhanced;
5) modification of a polynucleotide sequence encoding the polypeptide such that the polypeptide activity is enhanced (for example, modification of a polynucleotide sequence of the polypeptide gene so as to encode a polypeptide modified such that the activity of the polypeptide is enhanced);
6) introduction of a foreign polypeptide exhibiting the activity of the polypeptide or a foreign polynucleotide encoding the same;
7) codon optimization of a polynucleotide encoding the polypeptide;
8) selecting and modifying or chemically modifying an exposed site by analyzing a tertiary structure of the polypeptide; or
9) regulation of cellular localization of a protein (polypeptide); or
10) a combination of two or more selected from the above 1) to 9), but is not particularly limited thereto.

### More specifically,

The increase in the intracellular copy number of a polynucleotide encoding the polypeptide of 1) above may be achieved by the introduction into a host cell of a vector which is operably linked to the polynucleotide encoding the polypeptide and is capable of replicating and functioning independently of the host. Alternatively, it may be achieved by the introduction of one copy, or two or more copies of the polynucleotide encoding the polypeptide into a chromosome within the host cell. The introduction into the chromosome may be performed by introducing a vector capable of inserting the polynucleotide into the chromosome within the host cell into the host cell, but is not limited thereto. The vector is as described above.

The replacement of the gene expression regulatory region (or expression control sequence) on the chromosome encoding the polypeptide of 2) above with a sequence having strong activity may be, for example, the occurrence of a variation in the sequence through deletion, insertion, non-conservative or conservative substitution, or a combination thereof, or replacement with a sequence having stronger activity, so as to further strengthen the activity of the expression control region. The expression control region is not particularly limited thereto, but may include a promoter, an operator sequence, a sequence encoding a ribosome binding site, and a sequence regulating the termination of transcription and translation. As an example, it may be the replacement of the original promoter with a strong promoter, but is not limited thereto.

Examples of known strong promoters include, but are not limited to, CJ1 to CJ7 promoters (US Patent No. 7662943 B2), lac promoter, trp promoter, trc promoter, tac promoter, lambda phage PR promoter, PL promoter, tet promoter, gapA promoter, SPL7 promoter, SPL13(sm3) promoter (US Patent No. 10584338 B2), O2 promoter (US Patent No. 10273491 B2), tkt promoter, yccA promoter, and the like.

The modification of the nucleotide sequence encoding the start codon or the 5'-UTR region of the gene transcript encoding the polypeptide of 3) above may be, for example, substitution with a nucleotide sequence encoding another start codon having a higher polypeptide expression rate compared to the endogenous start codon, but is not limited thereto.

The modification of the amino acid sequence or the polynucleotide sequence of 4) and 5) above may be the occurrence of a variation in the sequence through deletion, insertion, non-conservative or conservative substitution of the amino acid sequence of the polypeptide or the polynucleotide sequence encoding the polypeptide, or a combination thereof, so as to enhance the activity of the polypeptide, or replacement with an amino acid sequence or polynucleotide sequence improved to have stronger activity or an amino acid sequence or polynucleotide sequence improved to have increased activity, but is not limited thereto. The replacement may specifically be performed by inserting the polynucleotide into the chromosome by homologous recombination, but is not limited thereto. The vector used at this time may further include a selection marker for confirming whether chromosomal insertion has occurred. The selection marker is as described above.

The introduction of an exogenous polynucleotide exhibiting the activity of the polypeptide of 6) above may be the introduction into a host cell of an exogenous polynucleotide encoding a polypeptide exhibiting the same/similar activity as the polypeptide. The exogenous polynucleotide is not limited in its origin or sequence as long as it exhibits the same/similar activity as the polypeptide. The method used for the introduction may be performed by a person skilled in the art appropriately selecting a known transformation method, and the polypeptide is produced by the expression of the introduced polynucleotide in the host cell, whereby its activity can be increased.

The codon optimization of the polynucleotide encoding the polypeptide of 7) above may be one in which the endogenous polynucleotide is codon-optimized to increase transcription or translation within the host cell, or one in which the codons of an exogenous polynucleotide are optimized so as to achieve optimized transcription and translation within the host cell.

The selecting, modifying, or chemically modifying an exposed site by analysis of the tertiary structure of the polypeptide of 8) above may be, for example, determining a template protein candidate according to the degree of sequence similarity by comparing the sequence information of the polypeptide to be analyzed with a database in which sequence information of known proteins is stored, and identifying the structure based thereon, then selecting, modifying, or modifying an exposed site to be modified or chemically modified.

The regulation of the intracellular localization of the protein (polypeptide) of 9) above may be targeting the protein (polypeptide) to a specific intracellular organelle or a specific intracellular space. For example, it may be targeting to the periplasm or cytoplasm through the addition or removal of a leader sequence functioning in the targeting of the protein (polypeptide), but is not limited thereto.

Such enhancement of polypeptide activity may be one in which the activity or concentration expression level of the corresponding polypeptide is increased based on the activity or concentration of the polypeptide expressed in a wild-type or pre-modification microorganism, or one in which the amount of a product produced from the polypeptide is increased, but is not limited thereto.

In the present disclosure, the term "microorganism (or strain)" may include both wild-type microorganisms and microorganisms in which natural or artificial genetic modification has occurred. The microorganism may be a microorganism in which a specific mechanism has been enhanced or weakened due to causes such as the insertion of an exogenous gene or the enhancement or weakening of the activity of an endogenous gene, and may be a microorganism comprising a genetic modification for the production of a target polypeptide, protein, or product (e.g., purine nucleotides and/or glycine). In the present application, the terms "microorganism," "strain," "host," and "host cell" may be used interchangeably.

The microorganism (or strain, recombinant cell) of the present disclosure may be a microorganism in which the activity of serine hydroxymethyltransferase is enhanced, which has the ability to produce (or production amount of) purine nucleotides and/or glycine, or in which the ability to produce purine nucleotides and/or glycine is improved (or increased).

In one example, the microorganism of the present disclosure may be, but is not limited to, a microorganism that naturally lacks the ability to produce purine nucleotides and/or glycine, or a microorganism that already possesses the ability to produce purine nucleotides and/or glycine, into which the polypeptide (variant) having serine hydroxymethyltransferase activity of the present disclosure or a polynucleotide encoding the same is introduced to confer or improve the ability to produce purine nucleotides.

In the present disclosure, the term "microorganism with enhanced serine hydroxymethyltransferase activity" may mean that a microorganism, which originally lacked the ability to produce purine nucleotides and/or glycine, has acquired the ability to produce purine nucleotides and/or glycine, or has acquired a higher ability to produce purine nucleotides and/or glycine than its original ability, by being engineered (mutated) to express the polypeptide (variant) having serine hydroxymethyltransferase activity described above.

In the present disclosure, "unmodified microorganism" does not exclude strains containing mutations that can occur naturally in microorganisms, and may refer to a wild-type strain or a natural strain itself, or a strain before its traits are changed by genetic variation due to natural or artificial factors. For example, according to one example, the unmodified microorganism may refer to a strain into which the polypeptide (variant) having serine hydroxymethyltransferase activity of the present application or a polynucleotide encoding the polypeptide (variant) having serine hydroxymethyltransferase activity has not been introduced, or a strain before such introduction. The "unmodified microorganism" may be used interchangeably with "strain before modification," "microorganism before modification," "unmutated strain," "unmodified strain," "unmutated microorganism," or "reference microorganism."

In the present disclosure, the reference microorganism may be a wild-type microorganism known to produce purine nucleotides, for example, *Corynebacterium stationis* ATCC 6872. Alternatively, the reference microorganism may be a microorganism known to produce purine nucleotides, for example, *Corynebacterium stationis* KCCM 12151P (US 2023-0192780 A1), but is not limited thereto. Additionally, the reference microorganism may be a wild-type microorganism known to produce glycine, for example, *Corynebacterium glutamicum* ATCC 13032, but is not limited thereto.

The microorganism producing purine nucleotides and/or glycine of the present disclosure is not particularly limited as long as it can produce purine nucleotides and/or glycine, but may be a microorganism of the genus *Corynebacterium*. The microorganism of the genus *Corynebacterium* may be at least one microorganism selected from the group consisting of *Corynebacterium stationis*, *Corynebacterium thermoaminogenes*, *Corynebacterium glutamicum*, *Brevibacterium flavum*, *Brevibacterium lactofermentum*, *Corynebacterium crudilactis*, *Corynebacterium deserti*, *Corynebacterium efficiens*, *Corynebacterium callunae*, *Corynebacterium singulare*, *Corynebacterium halotolerans*, *Corynebacterium striatum*, *Corynebacterium pollutisoli*, *Corynebacterium imitans*, *Corynebacterium testudinoris*, and *Corynebacterium flavescens*, but is not limited thereto. Specifically, the microorganism belonging to the genus *Corynebacterium* may be *Corynebacterium stationis* or *Corynebacterium glutamicum*, but is not limited thereto.

Another embodiment of the present disclosure provides a method for producing a purine nucleotide and/or glycine, comprising the step of culturing a microorganism in which the activity of serine hydroxymethyltransferase is enhanced in a medium; . Specifically, as another example of the present disclosure, provided is a method for producing a purine nucleotide or glycine, comprising the step of culturing the above-described microorganism comprising at least one selected from the group consisting of the above-described polypeptide, the above-described polynucleotide, and a vector comprising the above-described polynucleotide in a medium.

The above-described polypeptide, the above-described polynucleotide and the above-described vector, the microorganism in which the activity of the serine hydroxymethyltransferase is enhanced, the purine nucleotide, and the glycine are as described above.

In the present disclosure, "culturing" means growing a microorganism into which a polypeptide having serine hydroxymethyltransferase activity or a gene encoding the same of the present disclosure has been introduced or whose activity has been enhanced, for example, a microorganism of the genus of *Corynebacterium*, under appropriately controlled environmental conditions. The culturing process of the present application may be carried out according to a suitable medium and culturing conditions known in the art. Such a culturing process may be easily adjusted and used by those skilled in the art depending on the selected strain. Specifically, the culturing may be batch, continuous, and/or fed-batch, but is not limited thereto.

In the present disclosure, "medium" means a substance in which nutrients required for culturing a microorganism into which a polypeptide having serine hydroxymethyltransferase activity or a gene encoding the same of the present disclosure has been introduced or whose activity has been enhanced, for example, a microorganism of the genus *Corynebacterium*, are mixed as main components, and it supplies nutrients, growth factors, and the like, including water indispensable for survival and development. Specifically, as for the medium and other culturing conditions used for culturing the microorganism of the present disclosure, any medium may be used without particular limitation as long as it is a medium used for culturing conventional microorganisms; however, the microorganism of the present disclosure may be cultured while controlling temperature, pH, etc., under aerobic conditions in a conventional medium containing an appropriate carbon source, nitrogen source, phosphorus source, inorganic compounds, amino acids, and/or vitamins.

In the present disclosure, the carbon source may include carbohydrates such as glucose, saccharose, lactose, fructose, sucrose, and maltose; sugar alcohols such as mannitol and sorbitol; organic acids such as pyruvic acid, lactic acid, and citric acid; amino acids such as glutamic acid, methionine, and lysine. In addition, natural organic nutrients such as starch hydrolysate, molasses (e.g., blackstrap molasses), rice bran, cassava, sugarcane residue, and corn steep liquor may be used, and specifically, carbohydrates such as glucose and sterilized pre-treated molasses (i.e., molasses converted into reducing sugars) and the like may be used, and various other carbon sources in appropriate amounts may be utilized without limitation. These carbon sources may be used alone or in a combination of two or more types, but are not limited thereto.

As the nitrogen source, inorganic nitrogen sources such as ammonia, ammonium sulfate, ammonium chloride, ammonium acetate, ammonium phosphate, ammonium carbonate, and ammonium nitrate; and organic nitrogen sources such as amino acids like glutamic acid, methionine, and glutamine, peptone, NZ-amine, meat extract, yeast extract, malt extract, corn steep liquor, casein hydrolysate, fish or a decomposition product thereof, and defatted soybean cake or a decomposition product thereof may be used. These nitrogen sources may be used alone or in a combination of two or more types, but are not limited thereto.

The phosphorus source may include monopotassium phosphate, dipotassium phosphate, or sodium-containing salts corresponding thereto. As the inorganic compounds, sodium chloride, calcium chloride, iron chloride, magnesium sulfate, iron sulfate, manganese sulfate, calcium carbonate, and the like may be used, and in addition, amino acids, vitamins, and/or appropriate precursors may be included. These components or precursors may be added to the medium in a batch or continuous manner. However, it is not limited thereto.

In addition, during the culturing of the microorganism of the present disclosure, the pH of the medium may be adjusted by adding compounds such as ammonium hydroxide, potassium hydroxide, ammonia, phosphoric acid, and sulfuric acid to the medium in an appropriate manner. Further, during the culturing, foam formation may be suppressed by using an antifoaming agent such as fatty acid polyglycol ester. Furthermore, in order to maintain the aerobic state of the medium, oxygen or oxygen-containing gas may be injected into the medium, or to maintain anaerobic and microaerobic states, gas may be injected without injection or nitrogen, hydrogen, or carbon dioxide gas may be injected, but it is not limited thereto.

In the culturing of the present disclosure, the culturing temperature may be maintained at 20 to 45°C, or 25 to 37°C, specifically 25 to 37°C, and culturing may be performed for about 10 to 160 hours, or about 20 to 120 hours, but it is not limited thereto.

Purine nucleotides and/or glycine produced by the culturing of the present disclosure may be secreted into the medium or retained within the cells.

The method for producing a purine nucleotide of the present disclosure may comprise a step of adding an enzyme into the medium or a step of adding a microorganism expressing the enzyme. For example, the method may further comprise, after the step of culturing a microorganism that produces XMP, a step of adding an enzyme that converts XMP into GMP or a microorganism expressing the enzyme, and/or a step of culturing the microorganism.

The method for producing a purine nucleotide and/or glycine of the present disclosure may further comprise a step of recovering the purine nucleotide and/or glycine from the cultured microorganism (e.g., a microorganism of the genus *Corynebacterium* genus), the medium according to the culturing (the medium in which culturing was performed), or both of these. The recovering step may be further comprised after the culturing step.

The recovering may be collecting the target purine nucleotide and/or glycine using a suitable method known in the art according to the culturing method of the microorganism of the present disclosure, for example, a batch, continuous, or fed-batch culturing method. For example, various chromatographies such as centrifugation, filtration, treatment with a crystallization protein precipitant (salting-out method), extraction, ultrasonic disruption, ultrafiltration, dialysis, molecular sieve chromatography (gel filtration), adsorption chromatography, ion exchange chromatography, and affinity chromatography, HPLC, or a combination of these methods may be used, and the target purine nucleotide and/or glycine may be recovered from the medium or the microorganism using a suitable method known in the art.

In addition, the method for producing a purine nucleotide and/or glycine of the present disclosure may additionally comprise a purification step. The purification may be performed using a suitable method known in the art. In one example, when the method for producing a purine nucleotide and/or glycine of the present disclosure includes both a recovery step and a purification step, the recovery step and the purification step may be performed continuously or non-continuously regardless of the order, or may be performed simultaneously or integrated into one step, but is not limited thereto.

Another embodiment of the present disclosure is to provide a composition for producing purine nucleotides and/or glycine, comprising a microorganism having enhanced activity of serine hydroxymethyltransferase, a medium in which the microorganism is cultured, or a combination thereof.

The microorganism having enhanced activity of serine hydroxymethyltransferase, the purine nucleotide, and the glycine are as described above.

Still another embodiment provides a use of the microorganism for producing purine nucleotides and/or glycine.

Still another embodiment provides a use of the microorganism for the preparation of a composition for producing purine nucleotides and/or glycine.

The composition of the present disclosure may further comprise any suitable excipient commonly used in a composition for producing purine nucleotides and/or glycine, and such an excipient may be, for example, a preservative, a wetting agent, a dispersing agent, a suspending agent, a buffering agent, a stabilizer, or an isotonic agent, and the like, but is not limited thereto.

### [ADVANTAGEOUS EFFECTS]

The present application relates to a variant having serine hydroxymethyltransferase activity, a microorganism comprising the same, a composition for producing purine nucleotide and/or glycine comprising the microorganism, and a method for producing purine nucleotide and/or glycine comprising a step of culturing the microorganism, and it is possible to produce purine nucleotides and/or glycine with high yield by culturing a microorganism of the genus *Corynebacterium* comprising the serine hydroxymethyltransferase variant of the present application.

### [MODE FOR INVENTION]

The present disclosure is described in more detail below through examples. However, the following examples are merely preferred embodiments intended to illustrate the present disclosure and are therefore not intended to limit the scope of the present disclosure. Furthermore, technical details not described in this disclosure can be readily understood and implemented by those skilled in the technical field of the present disclosure or similar technical fields.

### Example 1: Identification of mutations of enhanced serine hydroxymethyltransferase

To enhance the production ability of purine nucleotides by ensuring a sufficient supply of glycine, which is one of the main substrates of purine nucleotide biosynthesis, a mutation library of *glyA*, a gene encoding serine hydroxymethyltransferase, was constructed to enhance the activity of serine hydroxymethyltransferase, and enhanced mutations with increased IMP production ability were identified.

### Example 1-1: Construction of a vector containing glyA

In order to construct a *glyA* library, a recombinant vector containing *glyA* was first constructed. The plasmid pCR^{™}2.1 vector (Invitrogen, K202020) for insertion and replacement of genes within the *Corynebacterium* chromosome was used as a parental vector, and a plasmid for additionally inserting the *glyA* gene using the Pcj7 promoter (U.S. Patent No. 7,662,943 B2) was constructed to enhance the activity of serine hydroxymethyltransferase.

Chromosomal genes of the wild-type *Corynebacterium stationis* ATCC6872 strain were isolated using Intron's G-spin Total DNA extraction mini kit (Cat. No 17045) according to the protocol provided in the kit, and a *glyA* gene fragment was obtained through polymerase chain reaction using a primer pair of SEQ ID NO: 1 and SEQ ID NO: 2. In addition, to obtain the Pcj7 promoter, a Pcj7 gene fragment was obtained through polymerase chain reaction using p117-cj7-gfp (U.S. Patent No. 7,662,943 B2) as a template and a primer pair of SEQ ID NO: 3 and SEQ ID NO: 4. The conditions for the PCR method were denaturation at 9°C for 5 minutes, followed by 20 cycles of denaturation at 95°C for 30 seconds, annealing at 55°C for 30 seconds, and polymerization at 72°C for 2 minutes, and finally a polymerization reaction at 72°C for 5 minutes.

The gene fragment obtained above was cloned into linear pCR2.1 cleaved with BamHI restriction enzyme through a Gibson assembly (NEB) method to obtain pCR2.1-Pcj7_*glyA*. The Gibson assembly reaction (based on 20 µl) was carried out by mixing 1 µl of linearized pCR2.1 vector, 1 µl of Pcj7 PCR DNA, 3 µl of *glyA* PCR DNA, 10 µl of Gibson assembly master mix, and 5 µl of PCR grade water, and proceeding with the reaction at 50°C for 30 minutes.

The sequences of the primers used in Example 1-1 are shown in Table 1 below.

**[Table 1]**

| Name | Sequence (5'-3') | SEQ ID NO |
|---|---|---|
| *glyA*_F | CCCAACGAAAGGAAACACTCCATATGACTACCCAGAATTCTTC C | SEQ ID NO: 1 |
| *glyA*-R | ACTGGCGGCCGTTACTAGTGGGATCCTTAGACGATCTTCCAGT CTTCC | SEQ ID NO: 2 |
| Pcj7_F | CAAGCTTGGTACCGAGCTCGCATCACCACGAGTTCCTTCAGGC TA | SEQ ID NO: 3 |
| Pcj7-R | GAAGAATTCTGGGTAGTCATATGGAGTGTTTCCTTTCGTTGGG TACG | SEQ ID NO: 4 |

### Example 1-2: Construction of glyA mutant library

Based on the vector constructed in Example 1-1, a *glyA* mutant library was generated using the following method.

Specifically, an Error-Prone PCR technique was used to introduce random mutations into *glyA*. The reaction was carried out using pCR2.1-Pcj7_*glyA* constructed in Example 1-1 as a PCR template and the primer pair of SEQ ID NO: 1 and SEQ ID NO: 2, and PCR buffer conditions were set for the purpose of introducing 4.6 bp mutations per 1 kb, proceeding with reference to the supplier's manual (Diversify PCR Random Mutagenesis Kit, TAKARA). The Error-prone PCR reaction (based on 50 µl) was carried out with a composition of 36 µl of PCR grade water, 5 µl of 10X TITANIUM Taq buffer, 4 µl of 8 mM MnSO₄, 1 µl of 2 mM dGTP, 1 µl of 50X Diversify dNTP Mix, 1 µl of Primer mix, 1 µl of Template DNA, and 1 µl of TITANIUM Taq polymerase, and the conditions for the PCR method were denaturation at 94°C for 30 seconds, followed by 25 cycles of denaturation at 94°C for 30 seconds and polymerization at 68°C for 2 minutes, and followed by performing a polymerization reaction at 68°C for 1 minute. The *glyA* gene fragment, into which random mutations were expected to be introduced, was cloned into linear pCR2.1-Pcj7 cut with NdeI and BamHI restriction enzymes through the Gibson assembly (NEB) method to obtain pCR2.1-Pcj7_*glyA*(Mut).

### Example 1-3: Construction of Corynebacterium stationis strain library containing glyA mutant vector library

Using the pCR2.1-Pcj7_*glyA*(Mut) variant vector library constructed in Example 1-2, *Corynebacterium stationis* KCCM12151P (US 2023-0192780 A1) was transformed by electroporation, and then plated on a selection medium containing 25 mg/L of kanamycin to obtain 10,000 colonies of strains into which mutation genes were inserted, which were selected as a primary candidate group. The selected strain libraries were named KCCM12151P_*glyA*(library_1) to KCCM12151P_*glyA*(library_10,000), respectively.

In addition, for use as a control group for experiments, the pCR2.1-Pcj7_*glyA* vector was introduced into *Corynebacterium stationis* KCCM12151P in the same manner and named KCCM12151P_*glyA*(WT).

### Example 1-4: Evaluation of glyA library and selection of strains

The 10,000 colonies obtained in Example 1-3 were each inoculated into 350 µl of autoclaved seed medium and cultured with shaking in a 96-deep well plate using an incubator shaker (INFORS) at a temperature of 30°C and 1200 rpm for 96 hours to be used as a seed culture. After dispensing 290 µl of autoclaved fermentation medium into a 96-deep well plate, 50 µl of each seed culture was inoculated, and cultured with shaking for 112 hours under the same conditions as above.

In order to analyze the production amount of 5'-inosinic acid produced in the culture broth, 100 µl of the culture supernatant after the end of culture was transferred to a 96-well black microplate. Next, absorbance was measured using a Near-Infrared Spectrometer, and 22 colonies of mutant strains showing an absorbance increased by 5% or more compared to the absorbance of the KCCM12151P_*glyA*(WT) strain were selected. Other colonies showed absorbance similar to or decreased compared to the control.

For the 22 selected strains, the confirmation of 5'-inosinic acid production through absorbance measurement was repeatedly performed in the same manner as above, and one strain, KCCM12151P_*glyA*(library_3371), which showed significantly improved 5'-inosinic acid production ability compared to the KCCM12151P_*glyA*(WT) strain, was selected.

### Example 1-5: Identification of glyA mutations by gene sequencing

To identify the genetic mutations in the selected mutant strain from Example 1-4, PCR was performed on KCCM12151P_*glyA*(library_3371) using the primer pair of SEQ ID NO: 5 and SEQ ID NO: 6. The amplified product was sequenced and compared to the *glyA* gene of the KCCM12151P strain.

As a result, it was confirmed that the *glyA* gene of the KCCM12151P_*glyA*(library_3371) strain contained three types of amino acid mutations. Specifically, in the KCCM12151P_*glyA*(library_3371) strain, it was confirmed that in the *glyA* amino acid sequence represented by SEQ ID NO: 31, the 388th phenylalanine was substituted with cysteine, the 409th lysine was substituted with arginine, and the 412th alanine was substituted with valine (F388C, K409R, A412V).

The sequences of the primers used in Example 1-5 are shown in Table 2 below.

**[Table 2]**

| Name | Sequence (5'->3') | SEQ ID NO: |
|---|---|---|
| *glyA*-lib_CF | GAATACTGTGAACTTGCTGG | SEQ ID NO: 5 |
| *glyA*-lib_CR | TTCCGCTAGGGACTTCTCCA | SEQ ID NO: 6 |

### Example 2: Construction of strains introduced with glyA mutant and evaluation of 5'-inosinic acid production ability

### Example 2-1: Construction of recombinant vectors for introducing glyA mutation

To determine whether the three *glyA* mutations F388V, K409R, and A412V identified in Example 1-5 could lead to increased IMP production, vectors for introducing each mutation into the endogenous *glyA* gene of the *Corynebacterium stationis* strain were constructed.

Specifically, using the plasmid pDC24 (SEQ ID NO: 51, Korean Patent Publication 10-2024-0167588A) for insertion and replacement of genes within the *Corynebacterium stationis* strain chromosome, vectors were constructed as follows.

Using gDNA (genomic DNA) of wild-type *Corynebacterium stationis* ATCC6872 as a template, PCR was performed using a pair of primers of SEQ ID NO: 7 and SEQ ID NO: 8 and a pair of primers of SEQ ID NO: 9 and SEQ ID NO: 10, respectively. PCR was performed at 94°C for 5 minutes for denaturation, followed by repeating 94°C for 30 seconds, 55°C for 30 seconds, and 72°C for 1 minute 30 times, and then at 72°C for 5 minutes. The pDC24 vector was treated with SmaI, and the PCR products obtained above were fusion-cloned using an In-Fusion^{®} HD cloning kit (Clontech). The plasmid obtained as a result of the cloning was named pDC24-*glyA*(F388C).

In addition, PCR and cloning were proceeded in the same manner using a primer pair of SEQ ID NO: 7 and SEQ ID NO: 11, and a primer pair of SEQ ID NO: 12 and SEQ ID NO: 10. The plasmid obtained as a result of the cloning was named pDC24-*glyA*(K409R).

In addition, PCR and cloning were proceeded in the same manner using a primer pair of SEQ ID NO: 7 and SEQ ID NO: 13, and a primer pair of SEQ ID NO: 14 and SEQ ID NO: 10. The plasmid obtained as a result of the cloning was named pDC24-*glyA*(A412V).

Finally, PCR and cloning were proceeded in the same manner using a primer pair of SEQ ID NO: 7 and SEQ ID NO: 8, a primer pair of SEQ ID NO: 9 and SEQ ID NO: 15, and a primer pair of SEQ ID NO: 16 and SEQ ID NO: 10. The plasmid obtained as a result of the cloning was named pDC24-*glyA*(F388C, K409R, A412V).

The sequences of the primers used in Example 2-1 are shown in Table 3 below.

**[Table 3]**

| NAME | SEQUNECE (5'->3') | SEQ ID NO |
|---|---|---|
| *glyA*(M3)_LF | AATTCGAGCTCGGTACCCACTACGGTGGATGT GAGCAC | SEQ ID NO: 7 |
| *glyA*(F388C)_R | GGTGcATGCTTCTGCATCCAGGCCGCGGGTAGC CA | SEQ ID NO: 8 |
| *glyA*(F388C)_F | CTGGATGCAGAAGCATgCACCGAGGTGGCAGA CAT | SEQ ID NO: 9 |
| *glyA*(M3)_RR | CGACTCTAGAGGATCCCCCGTCAGCATCGATG GTTTCC | SEQ ID NO: 10 |
| *glyA*(K409R)_R | GCGTGCGCGCAGCcTCTGGGTATCGGCGTTCTT GC | SEQ ID NO: 11 |
| *glyA*(K409R)_F | CGCCGATACCCAGAgGCTGCGCACGCGTGG ACA | SEQ ID NO: 12 |
| *glyA*(A412V)_R | AAGCTTGTCCACGCGTaCGCGCAGCTTCTGGGT AT | SEQ ID NO: 13 |
| *glyA*(A412V)_F | AAGCTGCGCGtACGCGTGGACAAGCTTGCTGA GCA | SEQ ID NO: 14 |
| *glyA*(K409R, A412V)_R | TGTCCACGCGTaCGCGCAGCcTCTGGGTATCGG CG | SEQ ID NO: 15 |
| *glyA*(K409R, A412V)_F | CAGAgGCTGCGCGtACGCGTGGACAAGCTTGCT GA | SEQ ID NO: 16 |

### Example 2-2: Construction of strains producing 5'-inosinic acid, into which glyA mutation are introduced

In order to assess the effect of F388C, K409R, A412V of *glyA* identified in Example 1-5, or combination mutations thereof, on IMP production ability, four types of strains in which each mutation was introduced into the endogenous *glyA* gene of the *Corynebacterium stationis* strain were constructed and evaluated.

After transforming *Corynebacterium stationis* KCCM12151P, a strain having 5'-inosinic acid production ability, with the pDC24-*glyA*(F388C), pDC24-*glyA*(K409R), pDC24-*glyA*(A412V), and pDC24-*glyA*(F388C, K409R, A412V) vectors constructed in Example 2-1 by electroporation, strains in which the mutation gene and the vector were inserted together on the chromosome were selected as a primary candidate group on a selection medium containing 25 mg/L of kanamycin. Thereafter, strains in which each mutation of the target gene was introduced were finally selected through gene sequence analysis using PCR using a primer pair of SEQ ID NO: 17 and SEQ ID NO: 18 in the strains where homologous recombination occurred. The finally selected strains were named CJI-3416(KCCM12151P_*glyA*(F388C)), CJI-3417(KCCM12151P_*glyA*(K409R)), CJI-3418(KCCM12151P_*glyA*(A412V)), and CJI-3419(KCCM12151P_*glyA*(F388C, K409R, A412V)), respectively.

The sequences of the primers used in Example 2-2 are shown in Table 4 below.

**[Table 4]**

| NAME | SEQUNECE (5'->3') | SEQ ID NO |
|---|---|---|
| *glyA*_seq_F | GCGGACCTGCGCAATTCTGA | SEQ ID NO: 17 |
| *glyA*_seq_R | CAGAGGATGCGGATAAGCAG | SEQ ID NO: 18 |

### Example 2-3: Evaluation of 5'-inosinic acid production ability of strains producing 5'-inosinic acid, into which glyA mutant are introduced

In order to measure the 5'-inosinic acid production ability of the strains CJI-3416(KCCM12151P_*glyA*(F388C)), CJI-3417(KCCM12151P_*glyA*(K409R)), CJI-3418(KCCM12151P_*glyA*(A412V)), and CJI-3419(KCCM12151P_*glyA*(F388C, K409R, A412V)) constructed in Example 2-2, flask titer evaluation was performed in the following manner.

Specifically, *Corynebacterium stationis* KCCM12151P, CJI-3416, CJI-3417, CJI-3418, and CJI-3419 were inoculated into 14 ml tubes containing 2.5 ml of the following seed medium and cultured with shaking at 30°C for 24 hours at 170 rpm. Then, 2 ml of the seed culture was inoculated into a 250 ml corner-baffle flask containing 29 ml of the following production medium (24 ml of main medium + 5 ml of separate sterilization medium) and cultured with shaking at 30°C for 72 hours at 170 rpm. After the end of culture, the production amount of 5'-inosinic acid was measured by the following method using HPLC (UC11-SIMAZU), and the OD value was measured by the following method.

Specifically, inosine-5-monophosphate disodium salt hydrate (Aldrich, 57510-5G) was used as a standard reagent used for HPLC at a concentration of 1 g/L. As a mobile phase for analysis, 10 g of 0.2%-Ammonium Dihydrogenphosphate, 1 g of 0.02%-Tetrabutylammonium phosphate monobasic, and 108 ml of 2.1% ACETONITRILE in 5 L of distilled water were used after correcting to pH 2.4 with H3PO4. The temperature of the column was 40°C, the flow rate was 1.0 ml/min, and 2 µl of the 20-fold diluted cultured sample was injected to measure the production amount of IMP. In addition, the culture-finished sample was diluted 100-fold with DW, and OD (Optical Density) was measured at a wavelength of 562 nm using a spectrophotometer (Eppendorf).

### <Seed medium for IMP production>

Glucose 1%, Peptone 1%, Meat extract 1%, Yeast extract 1%, Sodium chloride 0.25%, Adenine 100 mg/L, Guanine 100 mg/L, pH 7.2

### <Production medium for IMP production (Main medium)>

Sodium glutamate 0.1%, Ammonium chloride 1%, Magnesium sulfate 1.2%, Calcium chloride 0.01%, Iron sulfate 20 mg/L, Manganese sulfate 20 mg/L, Zinc sulfate 20 mg/L, Copper sulfate 5 mg/L, L-cysteine 23 mg/L, Beta-alanine 24 mg/L, Nicotinic acid 8 mg/L, Biotin 45 µg/L, Thiamine hydrochloride 5 mg/L, Adenine 30 mg/L, Phosphoric acid (85%) 1.9%, Glucose 4.2%, Fructose 2.4%

### <Production medium for IMP production (Separate sterilization medium)>

Phosphoric acid (85%) 23.3 g/L, Ammonia (28%) 16.25 g/L, Potassium hydroxide 25.5 g/L

Culture results according to the introduction of *glyA* mutations in the IMP producing strain of *Corynebacterium stationis* KCCM12151P are shown in Table 5 below. The above experiment was repeated 3 times, and the average value of the analysis results is shown. The residual sugar concentration is the result of measuring the residual amount of glucose at 48 hours out of a total culture time of 72 hours, based on 40 g of initial glucose.

**[Table 5]**

| Confirmation of 5'-inosinic acid production according to the introduction of *glyA* gene mutation | | | | |
|---|---|---|---|---|
| Strain name | Introducing type | OD | Concentration of residual sugar (g/L) | 5'- inosinic acid (g/L) |
| KCCM12151P | Control | 41.2 | 18.2 | 4.9 |
| CJI-3416 | *glyA*(F388C) | 41.1 | 17.1 | 5.1 |
| CJI-3417 | *glyA*(K409R) | 42.1 | 17.5 | 5.2 |
| CJI-3418 | *glyA*(A412V) | 40.9 | 17.3 | 5.0 |
| CJI-3419 | *glyA*(F388C, K409R, A412V) | 42.2 | 15.1 | 5.6 |

As a result, as shown in Table 5, the CJI-3416, CJI-3417, and CJI-3418 strains introduced with the F388C, K409R, or A412V mutations of *glyA* showed improved sugar consumption with residual sugar contents decreased by 6%, 4%, and 5%, respectively, compared to the control strain, and it was confirmed that IMP production amounts increased by 4%, 6%, and 2%, respectively. In the case of CJI-3419, which is a strain introduced with all three types of mutations, the residual sugar amount was decreased by 17% and the IMP production amount was improved by 14%, confirming that there was the greatest effect on improving IMP concentration when all F388C, K409R, and A412V mutations are included.

### Example 3: Construction of glyA-introduced strains and evaluation of glycine production ability

### Example 3-1: Construction of recombinant vectors for introducing glyA library mutations

In order to assess whether the three types of mutations F388C, K409R, and A412V of *glyA* identified in Example 1 could lead to an increase in glycine productivity of a strain of the genus *Corynebacterium*, mutation introduction was proceeded into the endogenous *glyA* gene of a *Corynebacterium glutamicum* strain. A total of 4 vectors were constructed to confirm the individual mutation effect and the integrated effect of the 3 types of mutations.

Specifically, they were constructed as follows using the plasmid pDC24 for insertion and replacement of genes within the *Corynebacterium* chromosome.

Using gDNA (genomic DNA) of wild-type *Corynebacterium glutamicum* ATCC13032 as a template, PCR was performed using a pair of primers of SEQ ID NO: 19 and 20 and a pair of primers of SEQ ID NO: 21 and 22, respectively. PCR was performed denaturation at 94°C for 5 minutes, followed by 30 cycles of denaturing at 94°C for 30 seconds, annealing at 55°C for 30 seconds, and polymerizing at 72°C for 1 minute, and then polymerization at 72°C for 5 minutes. The pDC24 vector was treated with SmaI and the PCR products obtained above were fusion-cloned. For fusion cloning, an In-Fusion^{®} HD cloning kit (Clontech) was used. The resulting plasmid was named pDC24-*glyA*(F389C).

In addition, PCR and cloning were proceeded in the same manner using a primer pair of sequences of SEQ ID NO: 19 and 23, and a primer pair of sequences of SEQ ID NO: 22 and 24. The resulting plasmid was named pDC24-*glyA*(S410R).

In addition, PCR and cloning were proceeded in the same manner using a primer pair of sequences of SEQ ID NO: 19 and 25, and a primer pair of sequences of SEQ ID NO: 22 and 26. The resulting plasmid was named pDC24-*glyA*(G413V).

Finally, PCR and cloning were proceeded in the same manner using a primer pair of sequences of SEQ ID NO: 19 and 20, a primer pair of sequences of SEQ ID NO: 21 and 27, and a primer pair of SEQ ID NO: 22 and 28. The resulting plasmid was named pDC24-*glyA*(F389C, S410R, G413V).

The sequences of the primers used in Example 3-1 are shown in Table 6 below.

**[Table 6]**

| Name | Sequence (5'->3') | SEQ ID NO: |
|---|---|---|
| *glyA*(M3)_LF | AATTCGAGCTCGGTACCCGATGATCGCGTCT GAGAACTT | SEQ ID NO: 19 |
| *glyA*(F389C)_R | TGCAACCTCAGTGcATGCAGGAATATCGAA | SEQ ID NO: 20 |
| *glyA*(F389C)_F | TTCGATATTCCTGCATgCACTGAGGTTGCA | SEQ ID NO: 21 |
| *glyA*(M3)_RR | CGACTCTAGAGGATCCCCCTTTAAGCTCATA ACCAACGT | SEQ ID NO: 22 |
| *glyA*(S410R)_R | TACACGGCCACGCAGAcgCTCAATGTCTGCG GA | SEQ ID NO: 23 |
| *glyA*(S410R)_F | TCCGCAGACATTGAGcgTCTGCGTGGCCGTGT A | SEQ ID NO: 24 |
| *glyA*(G413V)_R | AGCTTTGCTACACGGaCACGCAGAGACTCAA | SEQ ID NO: 25 |
| *glyA*(G413V)_F | TTGAGTCTCTGCGTGtCCGTGTAGCAAAGCT | SEQ ID NO: 26 |
| *glyA*(S410R, G413V)_R | GCTTTGCTACACGGaCACGCAGAcgCTCAATG TCTGC | SEQ ID NO: 27 |
| *glyA*(S410R, G413V)_F | GCAGACATTGAGcgTCTGCGTGtCCGTGTAGC AAAGC | SEQ ID NO: 28 |

### Example 3-2: Construction of glycine-producing strains introduced with glyA library mutations

In order to confirm the effect of the *glyA* F388C, K409R, and A412V mutations identified in Example 1 on glycine production ability, four types of strains in which mutations were introduced into the endogenous *glyA* gene of the *Corynebacterium glutamicum* strain were constructed and evaluated.

After transforming *Corynebacterium glutamicum* ATCC13032, a strain having glycine production ability, with the pDC24-*glyA*(F389C), pDC24-*glyA*(S410R), pDC24-*glyA*(G413V), and pDC24-*glyA*(F389C, S410R, G413V) vectors constructed in Example 3-1 by electroporation, strains in which the mutation gene and the vector were inserted together on the chromosome were selected as a primary candidate group on a selection medium containing 25 mg/L of kanamycin. Thereafter, final confirmation was made through gene sequence analysis through PCR using a primer pair of SEQ ID NO: 29 and SEQ ID NO: 30 in the strains where homologous recombination occurred. The selected strains were named CJ-2005(Cgl13032_*glyA*(F389C)), CJ-2006(Cgl13032_*glyA*(S410R)), CJ-2007(Cgl13032_*glyA*(G413V)), and CJ-2008(Cgl13032_*glyA*(F389C, S410R, G413V)), respectively.

The sequences of the primers used for gene sequence analysis in Example 3-2 are shown in Table 7 below.

**[Table 7]**

| Name | Sequence (5'->3') | SEQ ID NO: |
|---|---|---|
| *glyA*_seq_F | CCCAGGTCAGCAGGGTGG | SEQ ID NO: 29 |
| *glyA*_seq_R | CTCAATGACGCCTGCACA | SEQ ID NO: 30 |

### Example 3-3: Evaluation of glycine production ability of strains introduced with glyA mutation genes

In order to measure the glycine production ability of the CJ-2005, CJ-2006, CJ-2007, and CJ-2008 strains constructed in Example 3-2, culture was performed in the following manner.

After inoculating the parent strain *Corynebacterium glutamicum* ATCC13032 and each of the 4 types of mutant strains into 250 ml corner-baffle flasks containing 25 ml of seed medium, they were cultured with shaking at 30°C for 20 hours at 200 rpm to obtain seed cultures. Thereafter, 1 ml of the seed culture was inoculated into a 250 ml corner-baffle flask containing 24 ml of the production medium, and cultured at 30°C for 72 hours at 200 rpm to produce glycine.

The compositions of the seed medium and the fermentation medium are as follows.

### <Activation medium>

Beef extract 5 g/L, Polypeptone 10 g/L, Yeast extract 5 g/L, Urea 2 g/L, NaCl 2.5 g/L, Agar 20 g/L, Glucose 10 g/L, 10N NaOH 100 µl/L

### <Seed medium>

Glucose (anhydrous glucose) 20 g/L, Polypeptone 10 g/L, Yeast extract 10 g/L, (NH4)2SO₄ 10 g/L, Urea 1.5 g/L, KH2PO₄ 5.2 g/L, K2HPO₄ 10.7 g/L, d-Biotin 1.8 mg/L, Thiamine-HCl 9 mg/L, CAPA 9 mg/L, NCA 60 mg/L, MgSO₄ 0.5 g/L

### <Production medium>

CaCO₃ 30 g/L, Sucrose 50 g/L, MgSO₄ 0.6 g/L, (NH₄)₂SO₄ 20 g/L, KH₂PO₄ 1 g/L, Yeast extract 5 g/L, d-Biotin 0.05 mg/L, Thiamine-HCl 0.1 mg/L, MnSO₄ 18 µg/L, FeSO₄ 18 µg/L, ZnSO₄ 0.9 µg/L, CuSO₄ 0.9 µg/L

After the completion of culture, the production amount of glycine was measured using high-performance liquid chromatography, and the glycine concentration in the culture broth for each tested strain is shown in Table 8 below.

**[Table 8]**

| Confirmation of glycine production according to the introduction of *glyA* gene mutations | | | |
|---|---|---|---|
| Strain Name | Introduction Type | OD | Glycine Concentration (mg/L) |
| ATCC13032 | Control | 72.0 | 110.0 |
| CJ-2005 | *glyA*(F389C) | 68.8 | 121.0 |
| CJ-2006 | *glyA*(S410R) | 64.4 | 126.5 |
| CJ-2007 | *glyA*(G413V) | 66.2 | 116.6 |
| CJ-2008 | *glyA*(F389C, S410R, G413V) | 66.3 | 132.0 |

As shown in Table 8 above, it was confirmed that the *Corynebacterium glutamicum* CJ-2005, CJ-2006, and CJ-2007 strains, which introduced the *glyA* mutations F389C, S410R, or G413V corresponding to the *glyA* mutations F388C, K409R, and A412V identified in Example 1 showed increased glycine production by 10%, 15%, and 6%, respectively, compared to the control strain *Corynebacterium glutamicum* ATCC13032. In the case of CJ-2008, a strain into which all three types of mutations were introduced, the glycine production was improved by 20%, confirming that the inclusion of all F389C, S410R, and G413V mutations has the greatest effect on improving glycine concentration.

From the above description, those skilled in the art to which the present disclosure pertains will be able to understand that the present disclosure may be implemented in other specific forms without changing its technical spirit or essential features. In this regard, it should be understood that the embodiments described above are illustrative in all respects and not restrictive. The scope of the present disclosure should be interpreted such that all changes or modifications derived from the meaning and scope of the claims to be described later and their equivalent concepts, rather than the above detailed description, are included in the scope of the present disclosure.

## Claims

1. A polypeptide having serine hydroxymethyltransferase activity, wherein in an amino acid sequence having at least 80% sequence identity to the amino acid sequence of SEQ ID NO: 31, an amino acid corresponding to the 388th residue is substituted with another amino acid; an amino acid corresponding to the 409th residue is substituted with another amino acid; an amino acid corresponding to the 412th residue is substituted with another amino acid; or a combination thereof.

2. The polypeptide according to claim 1, wherein the polypeptide comprises an amino acid sequence in which, in the amino acid sequence of SEQ ID NO: 31, an amino acid corresponding to the 388th residue is substituted with another amino acid, an amino acid corresponding to the 409th residue is substituted with another amino acid, an amino acid corresponding to the 412th residue is substituted with another amino acid, or a combination thereof.

3. The polypeptide according to claim 1, wherein the polypeptide comprises an amino acid sequence in which, in the amino acid sequence of SEQ ID NO: 41, an amino acid corresponding to the 389th residue is substituted with another amino acid, an amino acid corresponding to the 410th residue is substituted with another amino acid, an amino acid corresponding to the 413th residue is substituted with another amino acid, or a combination thereof.

4. The polypeptide according to claim 1, wherein in the amino acid sequence having at least 80% sequence identity to the amino acid sequence of SEQ ID NO: 31,
an amino acid corresponding to the 388th residue is phenylalanine;
an amino acid corresponding to the 409th residue is lysine or serine; and
an amino acid corresponding to the 412th residue is alanine or glycine.

5. The polypeptide according to claim 1, wherein in the amino acid sequence having at least 80% sequence identity to the amino acid sequence of SEQ ID NO: 31,
an amino acid corresponding to the 388th residue is substituted with cysteine;
an amino acid corresponding to the 409th residue is substituted with arginine; or
an amino acid corresponding to the 412th residue is substituted with valine.

6. The polypeptide according to claim 1, wherein the polypeptide comprises any one amino acid sequence selected from the group consisting of SEQ ID NOs: 32 to 35 and SEQ ID NOs: 42 to 45.

7. A polynucleotide encoding the polypeptide according to any one of claims 1 to 6.

8. The polynucleotide according to claim 7, wherein the polynucleotide comprises any one nucleic acid sequence selected from the group consisting of SEQ ID NOs: 37 to 40 and SEQ ID NOs: 47 to 50.

9. A recombinant vector comprising the polynucleotide according to claim 7.

10. A microorganism comprising at least one selected from the group consisting of the polypeptide according to any one of claims 1 to 6, a polynucleotide encoding the polypeptide, and a vector comprising the polynucleotide.

11. The microorganism according to claim 10, wherein the microorganism has an increased activity of producing purine nucleotides or glycine.

12. The microorganism according to claim 11, wherein the purine nucleotide is at least one selected from the group consisting of 5'-inosine monophosphate (IMP), 5'-xanthosine monophosphate (XMP), and 5'-guanosine monophosphate (GMP).

13. The microorganism according to claim 10, wherein the microorganism is a microorganism of the genus *Corynebacterium*.

14. The microorganism according to claim 13, wherein the microorganism is *Corynebacterium stationis* or *Corynebacterium glutamicum*.

15. A method for producing purine nucleotides or glycine, comprising culturing in a medium a microorganism comprising at least one selected from the group consisting of the polypeptide according to any one of claims 1 to 6, a polynucleotide encoding the polypeptide, and a vector comprising the polynucleotide.

16. The method according to claim 15, further comprising recovering purine nucleotides or glycine from the cultured microorganism, the medium, or both.

17. The method according to claim 15, wherein the purine nucleotide is at least one selected from the group consisting of 5'-inosine monophosphate, 5'-xanthosine monophosphate, and 5'-guanosine monophosphate.

18. A composition for producing purine nucleotides or glycine, comprising a microorganism comprising at least one selected from the group consisting of the polypeptide according to any one of claims 1 to 6, a polynucleotide encoding the polypeptide, and a vector comprising the polynucleotide
